# EUROPÄISCHE PATENTSCHRIFT

(11) **EP 1 210 322 B1**
(45) Veröffentlichungstag und Bekanntmachung des Hinweises auf die Patenterteilung: **09.02.2005**
(21) Anmeldenummer: 00926871.5
(22) Anmeldetag: 12.04.2000
(51) Int. Cl.: C07C 253/30, C07C 67/307, C07C 255/50, C07C 69/78

(54) **VERFAHREN ZUR SEITENKETTENBROMIERUNG VON IN 2'-STELLUNG SUBSTITUIERTEN 4-METHYLBIPHENYL-DERIVATEN**
METHOD FOR CARRYING OUT THE SIDE CHAIN BROMINATION OF 4-METHYL BIPHENYL DERIVATIVES SUBSTITUTED AT THE 2'-POSITION
PROCEDE DE BROMATION DE CHAINE LATERALE DE DERIVES DE 4-METHYLBIPHENYLE SUBSTITUES EN POSITION 2'

(30) Priorität: 15.04.1999 DE 19917025
(43) Veröffentlichungstag der Anmeldung: 05.06.2002
(73) Patentinhaber: Boehringer Ingelheim Pharma GmbH & Co.KG, 55218 Ingelheim am Rhein (DE)
(72) Erfinder: SCHNEIDER, Heinrich DI, verstorben (DE)
(86) Internationale Anmeldenummer: PCT/EP2000/003246
(87) Internationale Veröffentlichungsnummer: WO 2000/063164

(56) Entgegenhaltungen:
- EP-A- 0 553 879
- DATABASE WPI Section Ch, Week 199502 Derwent Publications Ltd., London, GB; Class B05, AN 1995-011768 XP002145992 & JP 06 298683 A (SANKYO CO LTD), 25. Oktober 1994 (1994-10-25)
- DATABASE WPI Section Ch, Week 199901 Derwent Publications Ltd., London, GB; Class B05, AN 1999-005199 XP002145993 & JP 10 279533 A (NITTO CHEM IND CO LTD), 20. Oktober 1998 (1998-10-20)

## Beschreibung

Die Erfindung betrifft ein im technischen Maßstab anwendbares Verfahren zur Seitenkettenbromierung von in 2'-Stellung substituierten 4-Methylbiphenyl-derivaten mit-N-Brom-Imiden oder -Amiden zu in 2'-Stellung substituierten 4-Brommethylbiphenyl-derivaten der allgemeinen Formel **I** 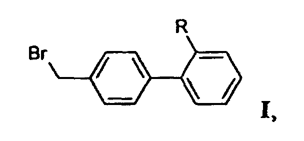 worin R die in der Beschreibung und in den Ansprüchen genannte Bedeutung aufweisen kann.

### HINTERGRUND DER ERFINDUNG

In 2'-Stellung substituierte 4-Brommethylbiphenyl-derivate der allgemeinen Formel **I** haben als Zwischenprodukte eine hohe Bedeutung bei der Herstellung von pharmazeutisch interessanten Wirkstoffen, insbesondere bei der Herstellung von Pharmawirkstoffen, die als Angiotensin-II-Antagonisten Verwendung finden können.

Verfahren zur Herstellung von Biphenylderivaten der allgemeinen Formel **I** sind aus dem Stand der Technik bekannt. Die EP-A-253310 schlägt zur Umsetzung von 4-Methyl-2'-methoxycarbonylbiphenyl zum entsprechenden Brommethyl-derivat die Verwendung von N-Bromsuccinimid (im Folgenden NBS) in Gegenwart des Radikalbildners Azo-bis-isobutyronitril (im Folgenden AIBN) in Tetrachlorkohlenstoff bei Siedetemperatur des Lösemittels vor. Die EP-A-553879 offenbart zur Herstellung von 4-Brommethylbiphenylderivaten eine Bromierung der entsprechenden Methyl-biphenyle mittels NBS oder N-Bromphthalimid in Gegenwart eines Radikalbildners in einem halogenierten Lösemittel. Die EP-A-595150 offenbart ein Herstellungsverfahren von beispielsweise 4-Brommethyl-2'-cyanobiphenyl durch Bromierung mit NBS in Gegenwart von Benzoylperoxid im Lösemittel Chlorbenzol. Die JP 06 298683 offenbart ein Verfahren zur Bromierung 2-substituierter Biphenyle zum entsprechenden Bromderivat unter Verwendung von NBS oder 1,3-Dibrom-5,5-dimethylhydantoin (im folgenden DDH) im großtechnischen Maßstab in einer photochemischen Reaktion.

Die aus dem Stand der Technik bekannten Verfahren zur Bromierung von in 2'-Stellung substituierten 4-Methylbiphenyl-Derivaten weisen bei der angestrebten großtechnischen Synthese allerdings eine Reihe von Nachteilen auf. So bedienen sie sich zumeist chlorierter Kohlenwasserstoffe als Lösemittel, welche bekanntermaßen Leber und Nieren schädigende Wirkungen haben können. Ferner, zeichnen sie sich bei der Umsetzung im technischen Maßstab zumeist durch lange Reaktionszeiten aus. Längere Reaktionszeiten führen jedoch zur verstärkten Bildung der Bis-Bromprodukte was sich nachteilig auf Ausbeute und Reinheit der erwünschten Mono-Brom-Derivate auswirkt.

Es ist daher Ziel und Aufgabe der vorliegenden Erfindung ein großtechnisch anwendbares Verfahren zur Herstellung von in 2'-Stellung substituierten 4-Brommethylbiphenyl-Derivaten bereitzustellen, welches die bei den aus dem Stand der Technik bekannten Verfahren auftretenden Nachteile überwindet.

### DETAILLIERTE BESCHREIBUNG DER ERFINDUNG

Überraschenderweise wurde gefunden, dass die vorstehend genannten Nachteile von aus dem Stand der Technik bekannten Bromierungsverfahren vermieden werden können, wenn als Lösemittel bei der Bromierung mittels N-Brom-Imiden oder -Amiden organische Carbonsäureester Verwendung finden.

Die vorliegende Erfindung betrifft folglich die Herstellung von in 2'-Stellung substituierten 4-Brommethyl-biphenyl-Derivaten der allgemeinen Formel **I** 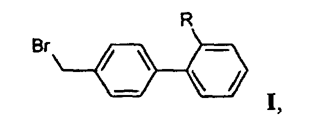 durch Umsetzung von in 2'-Stellung substituierten 4-Methylbiphenyl-Derivaten der allgemeinen Formel **II** 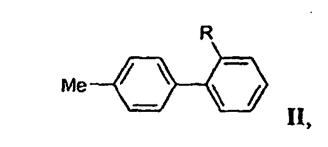 worin
- R: CN, COOH oder COO-tert-Butyl bedeutet,
dadurch gekennzeichnet, dass mittels NBS oder DDH in Gegenwart eines Radikalbildners ausgewählt aus AIBN oder Benzoylperoxid in Methylacetat bromiert wird

Zur Herstellung von in 2'-Stellung substituierten 4-Brommethyl-biphenyl-Derivaten der allgemeinen Formel **I** wird erfindungsgemäß wie folgt vorgegangen: In einem geeignet dimensionierten Reaktionsapparat werden pro 100 Mol Methylbiphenyl-Derivat der allgemeinen Formel **II** 100-120 Mol%, bevorzugt 100-110 Mol%, besonders bevorzugt ca. 105 Mol% Bromierungsreagenz, und gegebenenfalls 0 - 2 Mol% Radikalbildner, in das Lösemittel, den organischen Carbonsäureester, eingebracht und bis zum Sieden erhitzt. Im Verlauf der Reaktion (nach ca. 70-80 % der Reaktionszeit) geht das Bromierungsreagenz in Lösung. Sobald die Lösung praktisch farblos geworden ist, wird das Lösemittel größtenteils (ca. 50-90%) abdestilliert. Der verbleibende Rückstand wird in einem Gemisch aus einem zumindest teilweise wassermischbaren Lösemittel aufgenommen und das Produkt durch Zugabe von Wasser gefällt, wobei gleichzeitig das entstandene Imid oder Amid in Lösung geht. Bei säureempfindlichen Produkten kann durch Zugabe basischer Substanzen eine Zersetzung weitgehend vermieden werden. Überschüsse an Bromierungsreagenz können durch Zugabe reduzierender Substanzen desaktiviert werden. Nach Kristallisation wird das Produkt abgeschleudert, vorzugsweise mit dem gleichen Lösemittel/Wasser-Gemisch gewaschen und getrocknet.

Die Reaktionszeiten liegen üblicherweise in einem Bereich von 10 Minuten bis 8 Stunden. Es hat sich dabei herausgestellt, dass die jeweilige Reaktionszeit stark von der Reinheit der eingesetzten Verbindung der Formel **II** abhängig ist. Ferner hat sich gezeigt, dass die beobachteten Reaktionszeiten eine starke Konzentrationsabhängigkeit aufweisen. Seitens der Anmelderin wurden zur Durchführung der erfindungsgemäßen Syntheseverfahren pro 100 Mol Methylbiphenyl-Derivat der allgemeinen Formel **II** üblicherweise 30-100 l Carbonsäureester, bevorzugt 40-90 l Carbonsäureester, besonders bevorzugt etwa 50-80 l Carbonsäureester eingesetzt. Je nach eingesetzter Lösemittelmenge, die bei gegebenenfalls erwünschtem schnellerem oder langsamerem Reaktionsverlauf auch außerhalb der vorstehend genannten Grenzen liegen kann, ist es möglich, entscheidenden Einfluss auf die Reaktionsdauer zu nehmen.

Neben den bereits eingangs genannten Vorteilen des erfindungsgemäßen Verfahrens wie Verzicht auf halogenierte Lösemittel, kurze Reaktionszeiten sowie geringere Tendenz zur Bildung der Di-Bromaddukte ist das Verfahren durch eine hohe Raum-Zeit-Ausbeute gekennzeichnet.
Ferner wird durch die erfindungsgemäße Vorgehensweise eine verfahrenstechnisch wertvolle Verbesserung gegenüber anderen aus dem Stand der Technik bekannten Bromierungsverfahren mit N-Brom-Imiden oder -Amiden bereitgestellt. Da das erfindungsgemäß eingesetzte Lösemittel teilweise wassermischbar ist und die bei Bromierungen mit N-Brom-Imiden oder -Amiden entstehenden Imide oder Amide bei der erfindungsgemäßen Aufarbeitung in einem Gemisch aus Wasser und einem weiteren wassermischbaren Lösemittel in der Mutterlauge gelöst bleiben, ist ein verfahrenstechnisch aufwendiges Abfiltrieren derselben nicht mehr erforderlich.

Die nachfolgenden Beispiele dienen der Illustration exemplarisch durchgeführter, erfindungsgemäßer Syntheseverfahren zur Herstellung von in 2'-Stellung substituierten 4-Brommethylbiphenyl-Derivaten. Sie sind lediglich als mögliche, exemplarisch dargestellte Vorgehensweisen zu verstehen, ohne die Erfindung auf deren Inhalt zu beschränken.

### BEISPIEL 1

In einen 1200 l-Email-Rührwerksapparat werden 241,2 kg 4-Methylbiphenyl-2'-carbonsäuretert.-butylester, 168,2 kg NBS, 0,3 kg AIBN und 724 l Methylacetat eingebracht und das Gemisch auf 60 - 65°C erhitzt. Das Reaktionsgemisch beginnt zu sieden und nach etwa 80 % der Reaktionszeit geht das NBS in Lösung. Sobald die Lösung praktisch farblos geworden ist, werden unter Normaldruck 603 l Lösungsmittel abdestilliert und dann 482 l Isopropanol, 121 l Wasser und 14,8 kg Natriumacetat (wasserfrei) zugesetzt. Man kühlt auf 15 - 25°C ab und rührt noch 1 h nach. Das ausgefallene Produkt wird abgeschleudert, mit einem Gemisch aus 482 l Isopropanol und 121 l Wasser gewaschen und getrocknet.
- Ausbeute :: 250 kg 4-Brommethylbiphenyl-2'-carbonsäure-tert.-butylester
(80 % d. Th.)

### BEISPIEL 2

In einen 1200 l-Email-Rührwerksapparat werden 241,2 kg 4-Methylbiphenyl-2'-carbonsäuretert.-butylester, 168,2 kg NBS, 0,3 kg AIBN und 724 l Methylacetat eingebracht und das Gemisch auf 60 - 65°C erhitzt. Das Reaktionsgemisch beginnt zu sieden und nach etwa 80 % der Reaktionszeit geht das NBS in Lösung. Sobald die Lösung praktisch farblos geworden ist, werden unter Normaldruck 603 l Lösungsmittel abdestilliert und dann 482 l Aceton, 121 l Wasser und 14,8 kg Natriumacetat (wasserfrei) zugesetzt. Man kühlt auf 15 - 25°C ab und rührt noch 1 h nach. Das ausgefallene Produkt wird abgeschleudert, mit einem Gemisch aus 482 l Aceton und 121 l Wasser gewaschen und getrocknet.
- Ausbeute :: 250 kg 4-Brommethylbiphenyl-2'-carbonsäure-tert.-butylester
(80 % d. Th.)

### BEISPIEL 3

In einen 1200 l-Email-Rührwerksapparat werden 173,7 kg 4-Methyl-2'-cyanobiphenyl, 168,2 kg NBS und 695 l Methylacetat eingebracht und das Gemisch auf 60 - 65°C erhitzt. Das Reaktionsgemisch beginnt zu sieden und nach etwa 80 % der Reaktionszeit geht das NBS in Lösung. Sobald die Lösung praktisch farblos geworden ist, werden unter Normaldruck 608 l Lösungsmittel abdestilliert und dann 434 l Methanol und 174 l Wasser zugesetzt. Man kühlt auf 15 - 25°C ab und rührt noch 1 h nach. Das ausgefallene Produkt wird abgeschleudert, mit einem Gemisch aus 434 l Methanol und 174 l Wasser gewaschen und getrocknet.
- Ausbeute :: 205 kg 4-Brommethyl-2'-cyanobiphenyl
(83,7 % d. Th.)

### BEISPIEL 4

In einen 1200 l-Email-Rührwerksapparat werden 268,0 kg 4-Methylbiphenyl-2'-carbonsäuretert.-butylester, 157,3 kg DDH, 1,6 kg AIBN und 572 l Methylacetat eingebracht und das Gemisch auf 60 - 65°C erhitzt. Sobald die Lösung praktisch farblos geworden ist, werden unter Normaldruck 458 l Lösungsmittel abdestilliert und dann 530 l Isopropanol, 148 l Wasser und 16,4 kg Natriumacetat (wasserfrei) zugesetzt. Man kühlt auf 15 - 25°C ab und rührt noch 1 h nach. Das ausgefallene Produkt wird abgeschleudert, mit einem Gemisch aus 530 l Isopropanol und 148 l Wasser gewaschen und getrocknet.
- Ausbeute :: 295 kg 4-Brommethylbiphenyl-2'-carbonsäure-tert.-butylester
(85 % d. Th.)

### BEISPIEL 5

In einen 1200 l-Email-Rührwerksapparat werden 212 kg 4-Methylbiphenyl-2'-carbonsäure, 195,8 kg NBS, 1,6 kg AIBN und 530 l Methylacetat eingebracht und das Gemisch auf 60 - 65°C erhitzt. Das Reaktionsgemisch beginnt zu sieden und nach etwa 80 % der Reaktionszeit geht das NBS in Lösung. Sobald die Lösung praktisch farblos geworden ist, werden unter Normaldruck 424 l Lösungsmittel abdestilliert und dann 424 l Methanol und 106 l Wasser zugesetzt. Man kühlt auf 15 - 25°C ab und rührt noch 1 h nach. Das ausgefallene Produkt wird abgeschleudert, mit einem Gemisch aus 424 l Methanol und 106 l Wasser gewaschen und getrocknet.
- Ausbeute :: 240 kg 4-Brommethylbiphenyl-2'-carbonsäure (82,5 % d. Th.)

## Patentansprüche

1. Großtechnisches Verfahren zur Herstellung von in 2'-Stellung substituierten 4-Brommethyl-biphenyl-Derivaten der allgemeinen Formel (I) 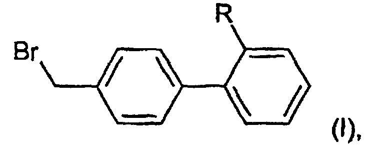 durch Umsetzung von in 2'-Stellung substituierten 4-Methylbiphenyl-Derivaten der allgemeinen Formel (II) 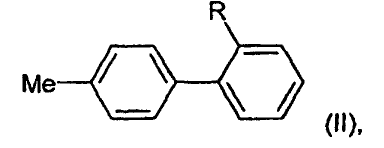 worin
R CN, COOH oder COO-tert-Butyl bedeutet,
**dadurch gekennzeichnet, daß** mittels N-Bromsuccinimid (NBS) oder N,N'-Dibrom-5,5-dimethylhydantoin (DDH) in Gegenwart eines Radikalbildners ausgewählt aus Azo-bis-isobutyronitril (AIBN) oder Benzoylperoxid in Methylacetat bromiert wird.

2. Verfahren gemäß Anspruch 1, wobei R COO-tert-Butyl bedeutet.

3. Verfahren gemäß einem der Ansprüche 1 oder 2, **dadurch gekennzeichnet, daß** bei der Aufarbeitung das entstandene Imid oder Amid in einem Gemisch aus Wasser und einem weiteren wassermischbaren Lösemittel in der Mutterlauge gelöst wird.

## Claims

1. Large-scale industrial process for producing 4-bromomethyl-biphenyl derivatives substituted in the 2' position of general formula (I) 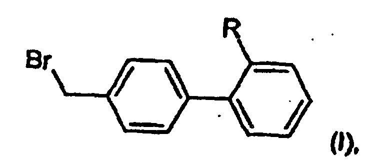 by reacting 4-methylbiphenyl derivatives substituted in the 2' position of general formula (II) 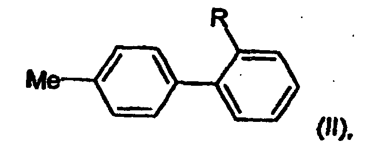 wherein
R denotes CN, COOH or COO-tert.butyl,
**characterised in that** bromination is carried out by means of N-bromosuccinimide (NBS) or N,N'-dibromo-5,5-dimethylhydantoin (DDH) in the presence of a radical former selected from among azo-bis-isobutyronitrile (AIBN) or benzoyl peroxide in methyl acetate.

2. Process according to claim 1, wherein R denotes COO-tert.butyl.

3. Process according to one of claims 1 or 2, **characterised in that** during the working up the imide or amide formed is dissolved in a mixture of water and another water-miscible solvent in the mother liquor.

## Revendications

1. Procédé industriel de production de dérivés de 4-bromométhylbiphényle substitués en position 2' de formule générale (I) 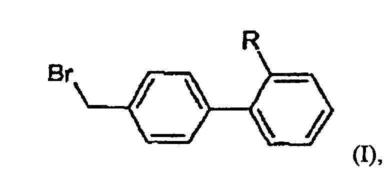 par réaction de dérivés de 4-méthylbiphényle substitués en position 2' de formule générale (II) 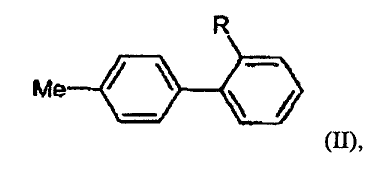 dans laquelle
R signifie CN, COOH ou COO-tert-butyl,
**caractérisé en ce que** l'on brome au moyen de N-bromosuccinimide (NBS) ou N,N'-dibromo-5,5-diméthylhydantoïne (DDH) en présence d'un formateur de radicaux choisi parmi l'azo-bis-isobutyronitrile (AIBN) ou le peroxyde de benzoyle dans de l'acétate de méthyle.

2. Procédé selon la revendication 1, dans lequel R signifie COO-tert-butyle.

3. Procédé selon l'une des revendications 1 ou 2, **caractérisée en ce que** pendant le traitement, l'imide ou l'amide résultant est dissous dans un mélange d'eau et d'un solvant miscible à l'eau supplémentaire dans la liqueur mère.
